# DEMANDE DE BREVET EUROPEEN

(11) **EP 1 930 763 A1**
(43) Date de publication de la demande: **11.06.2008**
(21) Numéro de dépôt: 07122166.7
(22) Date de dépôt: 03.12.2007
(51) Int. Cl.: G02B 27/10

(54) **Dispositif de multiplexage spatial de sources optiques**

(30) Priorité: 05.12.2006 FR 0610604
(71) Demandeur: THALES, 92200 Neuilly sur Seine (FR)
(72) Inventeur: Larat, Christian, 75006, PARIS (FR); Krakowski, Michel, 92340, BOURG LA REINE (FR)
(74) Mandataire: Esselin, Sophie

(57) **Abrégé**

L'invention concerne un dispositif optique de multiplexage comportant un ensemble de N sources de lumière (Si) émettant des faisceaux de lumière centrées sur des longueurs d'onde différentes dites centrales (λi) et de largeur spectrale (Δλi) caractérisé en ce qu'il comprend :
- une première voie comprenant des premiers moyens pour multiplexer en longueurs d'onde un premier sous-ensemble de sources, ladite première voie générant un premier faisceau de lumière présentant un premier état de polarisation ;
- une seconde voie comprenant des seconds moyens pour multiplexer en longueurs d'onde un second sous-ensemble de sources, ladite seconde voie générant un second faisceau de lumière présentant un second état de polarisation ;
- des troisièmes moyens pour multiplexer en polarisation les premier et second faisceaux de lumière et délivrer un troisième faisceau de lumière à grande largeur spectrale.

## Description

L'invention concerne un dispositif de multiplexage spatial de plusieurs sources optiques spectralement larges afin de superposer leurs faisceaux lumineux et ainsi disposer de sources de plus grande largeur spectrale. L'intérêt est d'obtenir une source de largeur spectrale plus grande avec la qualité spatiale propre à chaque source, pour des applications notamment de type OCT (Optical Coherent Tomography). Il s'agit de procédé d'imagerie non-invasive permettant le diagnostic médical. Cette technique est analogue à celle de l'échographie mais utilise un rayonnement lumineux à la place d'ultrasons, permettant une résolution nettement plus performante.

En effet, la résolution longitudinale de telles applications est inversement proportionnelle à la largeur spectrale de la source d'illumination utilisée. Usuellement, ces sources spectralement larges peuvent être des diodes superluminescentes, de plus fortes puissances que les diodes électroluminescentes. L'effet laser est empêché dans ces composants, d'où le spectre d'émission large.

Il a déjà été proposé des solutions utilisant le multiplexage en longueurs d'onde. Ce type de solution est notamment décrit dans la demande de brevet publiée n° 2 883 384. Il y est proposé un dispositif optique de multiplexage en longueur d'onde permettant de générer à partir d'une pluralité de faisceaux optiques émis par des sources différentes à des longueurs d'onde différentes, un faisceau de lumière unique polychromatique dans une direction commune. Plus précisément, il s'agit d'un dispositif optique de multiplexage en longueur d'onde compact comprenant plusieurs sources de lumière de type diodes laser comportant chacune un dispositif de sélection spectral de type transmissif disposé au voisinage des sources et permettant de sélectionner les longueurs d'onde des faisceaux d'émission en fonction de leur direction d'incidence sur un réseau de dispersion de façon que les directions des faisceaux diffractés soient toutes identiques, assurant ainsi le multiplexage des sources de lumière.

Les diodes laser peuvent être montées en barrette. Des optiques supplémentaires permettent de modifier les caractéristiques des faisceaux émis et notamment leur ellipticité.

Mais de telles solutions sont applicables à des sources substantiellement monochromatiques et non à des sources présentant une certaine largeur spectrale.

Dans ce contexte, la présente invention propose un dispositif optique dans lequel un multiplexage spectral combiné à un multiplexage en polarisation permettent de combiner plusieurs sources spectralement larges et décalées en fréquence les unes des autres pour obtenir une source présentant un spectre en fréquence global plus large, et donc une meilleure résolution longitudinale que celle obtenue avec une seule source. De plus, le dispositif proposé permet de conserver un taux de modulation du spectre global faible si tant est que les sources individuelles présente un spectre « lisse ». En effet, toute déviation par rapport au spectre idéal gaussien génère des bandes latérales dans le signal en retour pouvant réduire plus ou moins la résolution longitudinale effective. L'état de l'art antérieur ne permet pas de combiner plusieurs sources, avec une bonne efficacité et un bon recouvrement des spectres pour obtenir cet effet de lissage spectral.

Plus précisément, la présente invention a pour objet un dispositif optique de multiplexage comportant un ensemble de N sources de lumière Si émettant des faisceaux de lumière centrées sur des longueurs d'onde différentes dites centrales λi et de largeur spectrale Δλi caractérisé en ce qu'il comprend :
- une première voie comprenant des premiers moyens pour multiplexer en longueurs d'onde un premier sous-ensemble de sources, ladite première voie générant un premier faisceau de lumière présentant un premier état de polarisation ;
- une seconde voie comprenant des seconds moyens pour multiplexer en longueurs d'onde un second sous-ensemble de sources, ladite seconde voie générant un second faisceau de lumière présentant un second état de polarisation ;
- des troisièmes moyens pour multiplexer en polarisation les premier et second faisceaux de lumière et délivrer un troisième faisceau de lumière à grande largeur spectrale.

Avantageusement, la largeur spectrale d'une source Sᵢ est sensiblement égale à la moitié de la différence entre la longueur d'onde centrale λ ᵢ₊₁ d'une source S ᵢ₊₁ et la longueur d'onde centrale λ ᵢ₋₁ d'une source S ᵢ₋₁.

Selon une variante de l'invention, le premier état de polarisation est le même que le second état de polarisation et les troisièmes moyens comprennent également un système de rotation de polarisation pour mettre le premier et le second faisceau de lumière dans deux états de polarisation orthogonaux.

Selon une variante de l'invention, la largeur spectrale d'une source Sᵢ est sensiblement égale à la moitié de la différence entre la longueur d'onde centrale λ ᵢ₊₁ d'une source S ᵢ₊₁ et la longueur d'onde centrale λ ᵢ₋₁ centrale d'une source S ᵢ₋₁, lesdites longueurs d'onde étant classées en ordre croissant ou décroissant.

Selon une variante de l'invention, les sources présentant un ensemble discret de N longueurs d'onde centrales croissantes (λ₁,... , λᵢ, ... ,λ_{N}), le premier sous-ensemble comporte une première alternance de sources dites impaires centrées sur des longueurs d'onde dites impaires (λ₁,..., λ₂ᵢ₋₁, ...), le second sous-ensemble comportant une seconde alternance de sources dites paires centrées sur des longueurs d'onde dites paires (λ₂,... , λ₂ᵢ, ...).

Selon une variante de l'invention, les premiers moyens comprennent des premiers éléments optiques transparents pour une première série de sources dites impaires et réfléchissants pour une seconde série de sources impaires, les seconds moyens comprenant des seconds éléments optiques transparents pour une première série de sources dites paires et réfléchissants pour une seconde série de sources paires.

Selon une variante de l'invention, les premiers et les seconds moyens comprennent des composants optiques spécifiques présentant un coefficient de réflexion dépendant de la longueur d'onde.

Selon une variante de l'invention, les troisièmes moyens comprennent au moins un troisième élément optique transparent pour les sources dites impaires et réfléchissant pour les sources dites paires ou inversement.

Selon une variante de l'invention, les troisièmes moyens comprennent un composant anisotrope en surface ou en volume.

Selon une variante de l'invention, les faisceaux sont propagés en espace libre.

Avantageusement, le dispositif comprend des moyens pour collimater lesdits faisceaux de lumière.

Selon une variante de l'invention, les faisceaux sont propagés en optique guidée.

Avantageusement, le dispositif comprend des coupleurs en longueur d'onde et un coupleur en polarisation.

Selon une variante de l'invention, les premiers moyens et/ou les seconds moyens comprennent en outre des isolateurs optiques permettant d'assurer une direction privilégiée de propagation des premier et/ou second faisceaux de lumière dans ledit dispositif optique.

Selon une autre variante de l'invention, le dispositif comprend un isolateur optique actif sur le troisième faisceau de lumière.

L'invention sera mieux comprise et d'autres avantages apparaîtront à la lecture de la description donnée à titre non limitatif qui va suivre et grâce aux figures annexées parmi lesquelles :
- la figure 1 schématise le spectre d'émission de deux sources optiques dites impaires et une source optique dite paire utilisées dans un premier exemple de dispositif selon l'invention;
- la figure 2 schématise un premier exemple de dispositif de l'invention comportant deux sources optiques dites impaires et une source optique dite paire ;
- la figure 3 illustre la courbe de réflexion d'un exemple d'élément optique utilisé dans les premiers et/ou les seconds moyens d'un dispositif selon l'invention ;
- la figure 4 illustre le spectre d'émission obtenu avec la superposition de l'invention des trois sources S₁, S₂ et S₃ ;
- la figure 5 schématise un exemple de dispositif selon l'invention comportant un nombre N de sources optiques dans une configuration dite successive ;
- la figure 6 schématise une voie de sources dites impaires dans une configuration dite arborescente et pouvant être utilisée dans un dispositif de l'invention ;
- la figure 7 illustre un exemple de dispositif selon l'invention comprenant des sources en espace libre ;
- la figure 8 schématise un exemple de multiplexage en longueurs d'onde pouvant être utilisé dans un dispositif selon l'invention en optique guidée.

De manière générale, le dispositif optique de multiplexage de l'invention permet de multiplexer astucieusement des sources de longueurs d'onde différentes pour obtenir en sortie une source de lumière de bonne transmission constante sur une grande largeur spectrale grâce à une combinaison de multiplexage en longueur d'onde et de multiplexage en polarisation.

Plus précisément, le dispositif comprend des premiers et seconds moyens permettant de multiplexer en parallèle des sous-ensembles de sources optiques de même état de polarisation, et des moyens permettant de multiplexer en polarisation, l'ensemble des sous-ensembles de manière à obtenir une source de grande largeur spectrale.

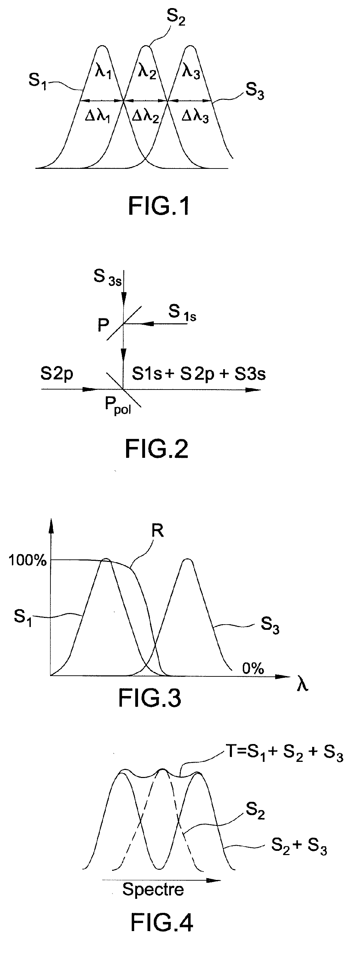

L'invention va être décrite dans le cas élémentaire de l'utilisation de trois sources mais peut être tout aussi bien appliquée dans le cas d'un plus grand nombre de sources de lumière. La figure 2 présente le principe de multiplexage dans le cas de sources représentées S₁, S₂, S₃ de largeur spectrale schématisées Δλ₁, Δλ₂, Δλ₃, de longueur d'onde centrale λ₁, λ₂, λ₃ respectivement et régulièrement espacées (λ₁ < λ₂ < λ₃), dont les spectres d'émission sont donnés en figure 1. Les sources dites impaires S₁ et S₃ présente un même état de polarisation s perpendiculaire au plan d'incidence, la source dite paire S₂ présentant un état de polarisation p dans le plan d'incidence. Sur le schéma de la figure 2, on a ainsi référencé les sources avec leur état de polarisation : S₁ₛ, S₃ₛ, S₂ₚ.

La première étape consiste à multiplexer en longueur d'onde les deux sources dites impaires spectralement les plus éloignées S₁ et S₃, à l'aide d'un système optique P. Il faut noter que les deux sources ont la même polarisation, si bien que le faisceau résultant reste linéairement polarisé. Dans le cas présenté, l'état de polarisation est l'état s. Le système optique P est par exemple une lame supportant un traitement diélectrique multicouche sur une de ses faces. Le coefficient de réflexion d'une telle couche en fonction de la longueur d'onde est représenté en trait plus gras par la courbe R sur la figure 3, conjointement avec les spectres d'émission des sources S₁ et S₃.

La deuxième étape consiste à multiplexer en polarisation les sources (S₁ + S₃) et S₂, avec le système optique Ppol. Ce dernier peut être par exemple une lame dichroïque spécifique, présentant usuellement une bonne transmission de la polarisation p et une bonne réflexion de la polarisation s (>95% dans les deux cas) et ce, sur une gamme spectrale très large. Le résultat du multiplexage est représenté par la courbe référencée T de la figure 4.

On obtient bien sur un seul faisceau la superposition des spectres initiaux des trois sources, avec une transmission du système global dans ce cas supérieure à 92%. Comme annoncé, le spectre est très lisse, mais est fortement dépendant du spectre réel de chaque source.

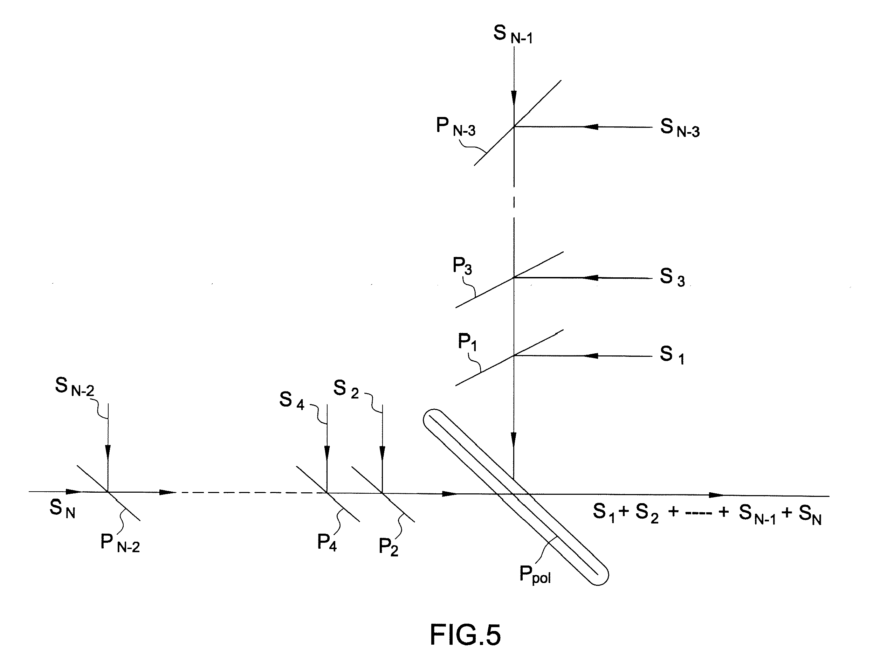

Plus de trois sources peuvent être multiplexées, la figure 5 illustre une configuration avec N sources S₁, S₂,..., S_{N}. La première étape consiste à multiplexer en longueur d'onde des séries de sources d'ordre impair d'une part à l'aide de plaques spécifiques P₁, P₃ ..., P_{N-1} et des sources d'ordre pair d'autre part à l'aide de plaques spécifiques P₂, P₄ ..., P_{N}. Par exemple, s'il y a N/2 sources impaires, N/2 -1 plaques P différentes sont utilisées, le multiplexage se faisant successivement. Dans ce cas, N/2 -1 plaques P sont également utilisées pour multiplexer les N/2 sources paires.

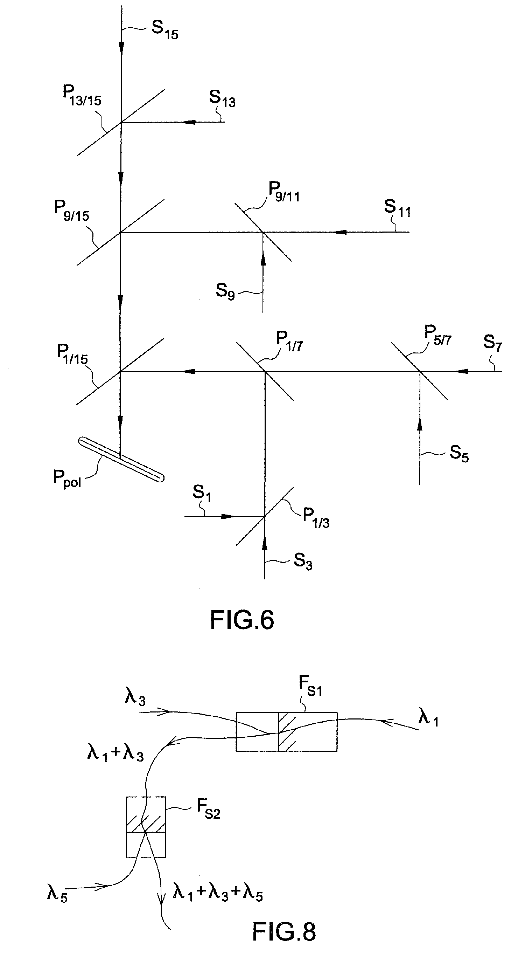

Il peut être envisagé soit une configuration dite successive comme celle illustrée en figure 5 soit une configuration dite arborescente comme celle illustrée en figure 6. Sur cette figure seule la branche concernant le premier faisceau et comportant les sources dites impaires est illustrée. En effet les deux branches peuvent avoir ou non le même type de configuration ; l'élément P_{a/b} combine les sources dont les indices extrêmes sont a et b, soit les plaques : P _{1/3}, P _{5/7}, P _{1/7}, P _{9/11}, P _{9/15}, P _{13/15} et P _{1/15} délivrant le faisceau multiplexé en longueur d'ondes dites impaires. Cet exemple illustre ainsi une configuration dite arborescente et comportant 8 sources impaires S₁, S₃, S₅, S₇, S₉, S₁₁, S₁₃ et S₁₅.

La dernière étape reste inchangée et consiste en un multiplexage en polarisation des deux ensembles de sources, paires et impaires par un élément Pₚₒₗ, éventuellement précédé d'un système pour tourner la polarisation d'un des deux ensembles si besoin.

### Exemple de réalisation en espace libre.

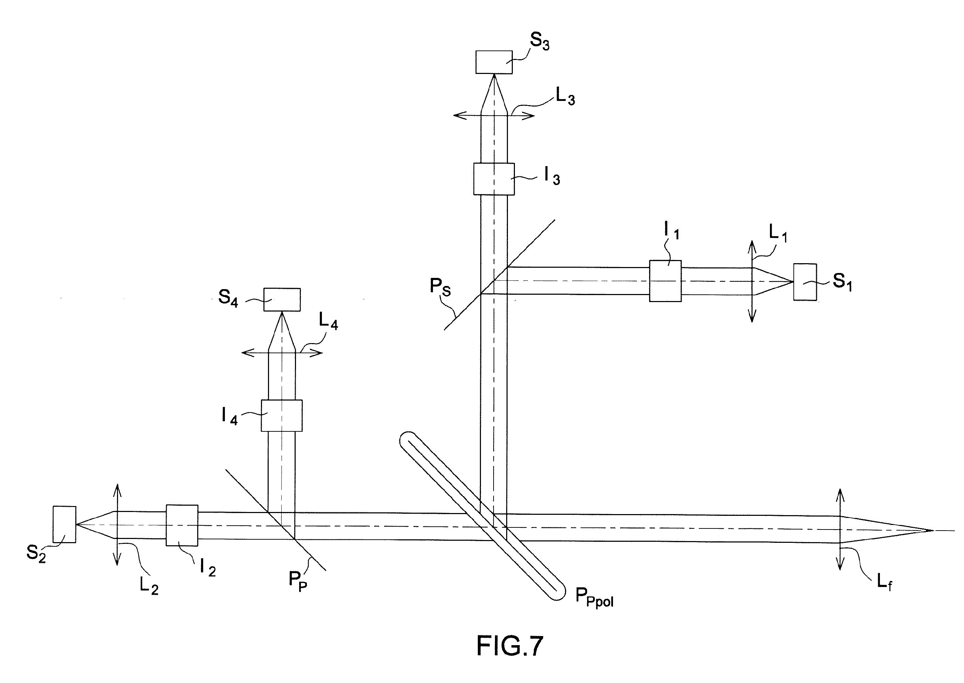

La figure 7 illustre un exemple de réalisation d'un dispositif de multiplexage comprenant quatre sources émettant des faisceaux en espace libre. Les sources S₁, S₂, S₃, S₄ sont avantageusement des diodes superluminescentes émettant des faisceaux de lumière centrées sur des longueurs d'onde différentes dites centrales pouvant typiquement être comprises entre 1 micron et 1,3 micron avec des largeurs Δλi typiquement de l'ordre 50 nanomètres. Les diodes sont orientées de sorte que la polarisation de S₁ et S₃ soit s pour Pₚₒₗ (i.e. perpendiculaire au plan du dessin) et celle de S₂ et S₃ p (parallèle au dessin). Les faisceaux de lumière sont collimatés grâce à des lentilles ou des ensembles de lentilles L₁, L₂, L₃, L₄ en direction des plaques Ps et Pp. Avantageusement des isolateurs optiques I₁, I₂, I₃, I₄ sont également prévus pour éviter des retours de lumière en direction des sources. Le faisceau de lumière composite S₁+S₂+S₃+S₄ peut être ensuite focalisé par une lentille Lf. Typiquement, les isolateurs peuvent être de type un rotateur de Faraday compris entre deux polariseurs.

Si les sources sont non polarisées les propriétés des lames Ps, Pp indépendantes de la polarisation, le faisceau en sortie est partagé en deux sur les deux voies de sortie de Pₚₒₗ.

### Exemple de réalisation en optique guidée

La figure 8 illustre un schéma de fibre optique comportant localement des éléments par exemple dichroïques permettant d'être sélectivement transmissif pour certaines longueurs d'onde et réfléchissant pour d'autres longueurs d'onde. Sur la figure 8, seuls des tronçons de fibres sont représentés avec leurs éléments spécifiques F_{S1}, F_{S2}, permettant de montrer le multiplexage en longueur d'onde respectivement pour une source S₁ de longueur d'onde centrale λ₁, une source S₃ de longueur d'onde centrale λ₃ et une source S₅ de longueur d'onde centrale λ₅. en introduisant à chaque fois deux sources à contre-sens afin de les sommer en longueur d'onde. Et ainsi de proche en proche il est possible de constituer les deux sous-ensembles de sources impaires et paires pour dans une dernière étape multiplexer en polarisation les deux sous-ensembles de sources fibrées. Avantageusement, les fibres optiques sont à maintien de polarisation. Si les sources sont non polarisées et/ou les fibres sans maintien de polarisation, le faisceau en sortie est partagé en deux sur les deux voies de sortie du coupleur en polarisation.

Selon une autre variante, le dispositif de l'invention combine les deux approches : une propagation libre et une propagation guidée, par exemple les premiers et seconds moyens en propagation guidée et les troisièmes moyens en propagation libre.

## Revendications

1. Dispositif optique de multiplexage comportant un ensemble de N sources de lumière (Si) émettant des faisceaux de lumière centrées sur des longueurs d'onde différentes dites centrales (λi) et de largeur spectrale (Δλi) **caractérisé en ce qu'**il comprend :
- une première voie comprenant des premiers moyens pour multiplexer en longueurs d'onde un premier sous-ensemble de sources, ladite première voie générant un premier faisceau de lumière présentant un premier état de polarisation ;
- une seconde voie comprenant des seconds moyens pour multiplexer en longueurs d'onde un second sous-ensemble de sources, ladite seconde voie générant un second faisceau de lumière présentant un second état de polarisation ;
- des troisièmes moyens pour multiplexer en polarisation les premier et second faisceaux de lumière et délivrer un troisième faisceau de lumière à grande largeur spectrale ;
- toute source présentant un spectre se recouvrant partiellement avec les spectres des sources qui lui sont adjacentes spectralement.

2. Dispositif optique selon la revendication 1, **caractérisé en ce que** le premier état de polarisation est le même que le second état de polarisation et **en ce que** les troisièmes moyens comprennent également un système de rotation de polarisation pour mettre le premier et le second faisceau de lumière dans deux états de polarisation orthogonaux.

3. Dispositif optique selon l'une des revendications 1 ou 2, **caractérisé en ce que** la largeur spectrale d'une source Sᵢ est sensiblement égale à la moitié de la différence entre la longueur d'onde centrale λ ᵢ₊₁ d'une source S ᵢ₊₁ et la longueur d'onde centrale λ ᵢ₋₁ d'une source Sᵢ₋₁, lesdites longueurs d'onde étant classées en ordre croissant ou décroissant.

4. Dispositif optique selon l'une des revendications 1 à 3, **caractérisé en ce que** les sources présentant un ensemble discret de N longueurs d'onde centrales croissantes ou décroissantes (λ₁,... , λᵢ, ... ,λ_{N}), le premier sous-ensemble comporte une première alternance de sources dites impaires centrées sur des longueurs d'onde dites impaires (λ₁,... , λ₂ᵢ₋₁, ...), le second sous-ensemble comportant une seconde alternance de sources dites paires centrées sur des longueurs d'onde dites paires (λ₂,... , λ₂ᵢ, ...).

5. Dispositif optique selon la revendication 4, **caractérisé en ce que** les premiers moyens comprennent des premiers éléments optiques transparents pour une première série de sources dites impaires et réfléchissants pour une seconde série de sources impaires, les seconds moyens comprenant des seconds éléments optiques transparents pour une première série de sources dites paires et réfléchissants pour une seconde série de sources paires.

6. Dispositif optique selon la revendication 5, **caractérisé en ce que** les premiers et les seconds moyens comprennent des composants optiques spécifiques présentant un coefficient de réflexion dépendant de la longueur d'onde.

7. Dispositif optique selon l'une des revendications 1 à 6, **caractérisé en ce que** les troisièmes moyens comprennent au moins un troisième élément optique transparent pour les sources dites impaires et réfléchissant pour les sources dites paires ou inversement.

8. Dispositif optique selon la revendication 7, **caractérisé en ce que** les troisièmes moyens comprennent un composant anisotrope en surface ou en volume.

9. Dispositif optique selon l'une des revendications 1 à 8, **caractérisé en ce que** les faisceaux sont propagés en espace libre.

10. Dispositif optique selon la revendication 9, **caractérisé en ce qu'**il comprend des moyens pour collimater lesdits faisceaux de lumière.

11. Dispositif optique selon l'une des revendications 1 à 8, **caractérisé en ce que** les faisceaux sont propagés en optique guidée.

12. Dispositif optique selon la revendication 11, **caractérisé en ce qu'**il comprend des coupleurs en longueur d'onde et un coupleur en polarisation.

13. Dispositif optique selon l'une des revendications 1 à 12, **caractérisé en ce que** les premiers moyens et/ou les seconds moyens comprennent en outre des isolateurs optiques permettant d'assurer une direction privilégiée de propagation des premier et/ou second faisceaux de lumière dans ledit dispositif optique.

14. Dispositif optique selon l'une des revendications 1 à 12, **caractérisé en ce qu'**il comprend un isolateur optique actif sur le troisième faisceau de lumière.
